# EUROPÄISCHE PATENTSCHRIFT

(11) **EP 0 749 419 B1**
(45) Veröffentlichungstag und Bekanntmachung des Hinweises auf die Patenterteilung: **24.10.2001**
(21) Anmeldenummer: 95910528.9
(22) Anmeldetag: 27.02.1995
(51) Int. Cl.: C07C 259/06, C07C 259/10, C07C 259/14, C07C 259/18, C07C 323/47, C07C 317/28, A01N 37/52

(54) **OXIM-DERIVATE UND IHRE VERWENDUNG ALS SCHÄDLINGSBEKÄMPFUNGSMITTEL**
OXIME DERIVATIVES AND THEIR USE AS PESTICIDES
DERIVES D'OXIME ET LEUR UTILISATION COMME PESTICIDES

(30) Priorität: 10.03.1994 DE 4408006; 29.04.1994 DE 4414986; 24.06.1994 DE 4422154
(43) Veröffentlichungstag der Anmeldung: 27.12.1996
(73) Patentinhaber: BAYER AG, 51368 Leverkusen (DE)
(72) Erfinder: GERDES, Peter, D-52080 Aachen (DE); GAYER, Herbert, D-40789 Monheim (DE); KRÜGER, Bernd-Wieland, D-51467 Bergisch Gladbach (DE); GALLENKAMP, Bernd, D-42113 Wuppertal (DE); DEHNE, Heinz-Wilhelm, D-53125 Bonn (DE); DUTZMANN, Stefen, D-40721 Hilden (DE); HÄNSSLER, Gerd, D-51381 Leverkusen (DE); STENZEL, Klaus, D-40595 Düsseldorf (DE)
(86) Internationale Anmeldenummer: EP9500708
(87) Internationale Veröffentlichungsnummer: WO9524383

(56) Entgegenhaltungen:
- EP-A- 0 039 168
- EP-A- 0 174 046
- EP-A- 0 273 432
- EP-A- 0 477 631
- WO-A-94/00422
- WO-A-94/14322
- WO-A-94/22844
- GB-A- 2 039 474
- CHEMICAL ABSTRACTS, vol. 120, no. 6, 7. Februar 1994, Columbus, Ohio, US; abstract no. 55101k, J.C. JUNG ET. AL. 'Model studies on the structure of poly(amideoximes) and their cyclodehydration reactions leading to poly(1,2,4-oxadiazoles).' Seite 7 ;Spalte 1 ; & J. POLYM. SCI. PART A. POLY. CHEM.,, Bd.31, Nr.3, 1993 Seiten 3351 - 9

## Beschreibung

Die Erfindung betrifft neue Oxim-Derivate, mehrere Verfahren zu ihrer Herstellung und ihre Verwendung als Schädlingsbekämpfungsmittel.

Es ist bekannt, daß verschiedene substituierte Alkoximino- und Alkoxymethylenacetamide fungizide Eigenschaften besitzen (vgl. z.B. EP-A 398 692, EP-A 468 775, DE-A 40 30 038 und WO-A 92/13 830).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue Oxim-Derivate der allgemeinen Formel (I) gefunden, 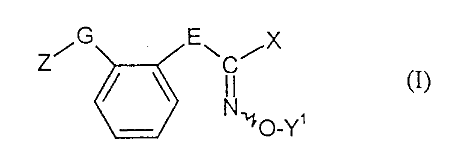 in welcher
- E: für eine direkte Bindung oder für eine Gruppierung der Formel 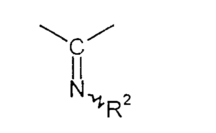 steht,
worin
R² für Methoxy steht,
- G: für eine Gruppe der Formel

-CH₂-O-,

oder

-O-CH₂-

steht,
- X: für die Gruppierungen -OX¹, -SX¹, -SOX¹, -SO₂X¹ oder -NX²X³ steht, wobei
- X¹, X² und X³: unabhängig voneinander für Wasserstoff, für gegebenenfalls durch Fluor, Chlor, Cyano, Hydroxy, Amino, Methoxy, Methylthio (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls durch Fluor, Chlor, Brom, Cyano, Carboxy, Phenyl (welches gegebenenfalls durch Fluor, Chlor, Methyl, Trifluormethyl oder Trifluormethoxy substituiert ist), Methyl, Ethyl, n- oder i-Propyl, Methoxycarbonyl oder Ethoxycarbonyl substituiertes Cyclopropyl, Cyclobutyl, Cyclopentyl oder Cyclohexyl,
für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy, oder
- X² und X³: zusammen einen gegebenenfalls wie oben substituierten Pyrazol-, Imidazol- oder Triazolring bilden,
- Y¹: für Wasserstoff, für gegebenenfalls durch Fluor, Chlor, Cyano, Hydroxy, Amino, Methoxy, Methylthio (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls durch Fluor, Chlor, Brom, Cyano, Carboxy, Phenyl (welches gegebenenfalls durch Fluor, Chlor, Methyl, Trifluormethyl oder Trifluormethoxy substituiert ist), Methyl, Ethyl, n- oder i-Propyl, Methoxycarbonyl oder Ethoxycarbonyl substituiertes Cyclopropyl, Cyclobutyl, Cyclopentyl oder Cyclohexyl,
für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy,
- Z: für gegebenenfalls substituiertes Phenyl oder gegebenenfalls substituiertes Pyridinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

Weiterhin wurde gefunden, daß man die neuen Oxim-Derivate der allgemeinen Formel (I) erhält, wenn man
a) Thiocarbonyl-Derivate der allgemeinen Formel (II), 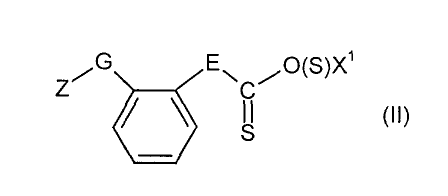 in welcher
   E, G, X¹ und Z die oben angegebene Bedeutung haben,
   mit Hydroxylamin-Derivaten der allgemeinen Formel (III),

   H₂N―O―Y¹ (III),

   in welcher
   - Y¹: die oben angegebene Bedeutung hat,
   oder mit deren Säureadditionssalzen gegebenenfalls in Gegenwart eines Verdünnungsmittels sowie gegebenenfalls in Gegenwart eines Reaktionshilfsmittels umsetzt;
   oder
b) Amid-Derivate der allgemeinen Formel (IV) 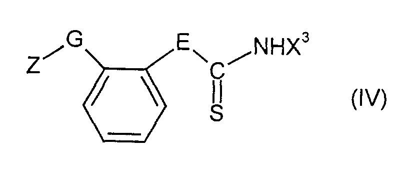 in welcher
   E, G, X³ und Z die oben angegebenen Bedeutungen haben,
   in üblicher Weise alkyliert und die so erhaltenen Imino-Derivate der Formel (IVa) 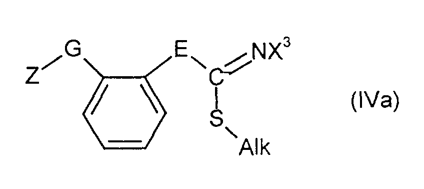 in welcher
   E, G, X³ und Z die oben angegebenen Bedeutungen haben und
   - Alk: für Alkyl, vorzugsweise Methyl steht,
   gegebenenfalls ohne Isolierung mit Hydroxylamin-Derivaten der allgemeinen Formel (III) oder mit deren Säureadditionssalzen gegebenenfalls in Gegenwart eines Verdünnungsmittels sowie gegebenenfalls in Gegenwart eines Reaktionshilfsmittels umsetzt;
   oder
c) Amid-Derivate der allgemeinen Formel (V) 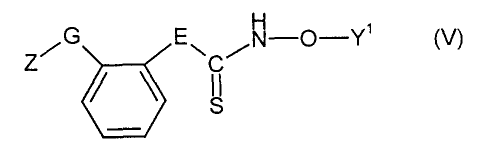 in welcher
   E, G, Y¹ und Z die oben angegebenen Bedeutungen haben,
   in üblicher Weise alkyliert und die so erhaltenen Oxime der Formel (Va) 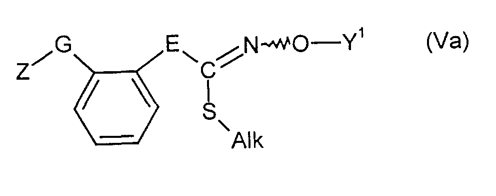 in welcher
   E, G, Y¹, Z und Alk die oben angegebenen Bedeutungen haben,
   gegebenenfalls ohne Isolierung mit Aminen der allgemeinen Formel (VI)

   HNX²X³ (VI)

   in welcher
   - X² und X³: die oben angegebenen Bedeutungen haben,
   gegebenenfalls in Gegenwart eines Verdünnungsmittels umsetzt.

Verbindungen der Formel (I) mit X=SOX¹ oder SO₂X¹ werden erhalten, indem man entsprechende Verbindungen der Formel (I) mit X=SX¹ in allgemein bekannter Weise oxidiert, wie z.B. durch Oxidation mittels Wasserstoffperoxid oder organischen Persäuren.

Schließlich wurde gefunden, daß die neuen Oxim-Derivate der allgemeinen Formel (I) sehr starke fungizide Wirksamkeit zeigen.

Die erfindungsgemäßen Verbindungen können gegebenenfalls als Mischungen verschiedener möglicher isomerer Formen, insbesondere von E- und Z-Isomeren, gegebenenfalls aber auch von optischen Isomeren und Diastereomeren vorliegen. Es werden sowohl die E- als auch die Z-Isomeren wie auch beliebige Mischungen der weiteren möglichen Isomeren beansprucht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise[2-(2-Methylphenoxymethyl)-phenyl]-2-methoximinothioessigsäuremethylester und O-Methyl-hydroxylammoniumchlorid als Ausgangsstoffe, so kann der Reaktionsablauf des erfindungsgemäßen Verfahrens (a) durch das folgende Formelschema skizziert werden:

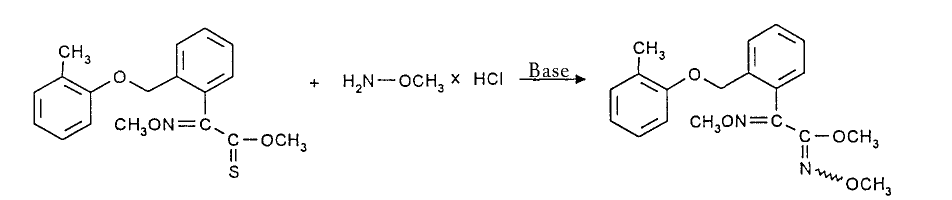

Verwendet man beispielsweise {2-[1-(3-Trifluormethylphenyl)-ethylidenaminoxymethyl]-phenyl}-2-methoximino-thioessigsäuremethylamidalsAusgangsstoff,Methyljodid als Alkylierungsmittel und O-Methylhydroxylammoniumchlorid als weiteren Ausgangsstoff, so kann der Reaktionsablauf des erfindungsgemäßen Verfahrens (b) durch das folgende Formelschema skizziert werden:

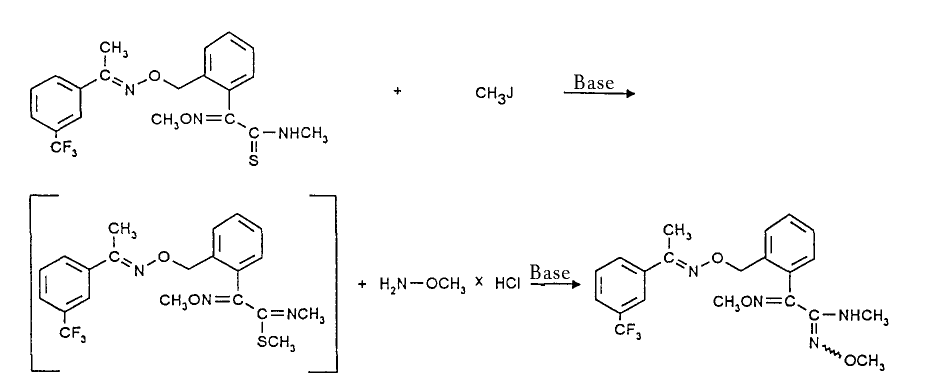

Verwendet man beispielsweise [2-(2-Methylphenoxymethyl)-phenyl]-2-methoximinothioessigsäure-methoxyamid als Ausgangsstoff, Methyljodid als Alkylierungsmittel und Dimethylamin als weiteren Ausgangsstoff, so kann der Reaktionsablauf des erfindungsgemäßen Verfahrens (c) durch das folgende Formelschema skizziert werden:

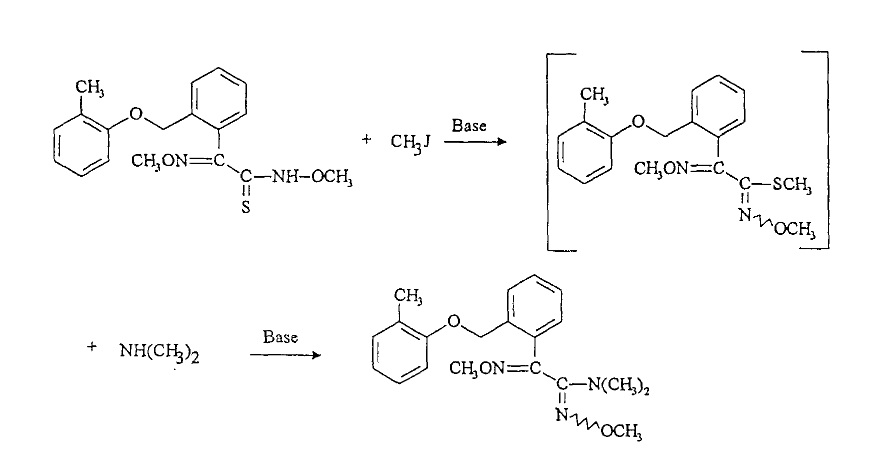

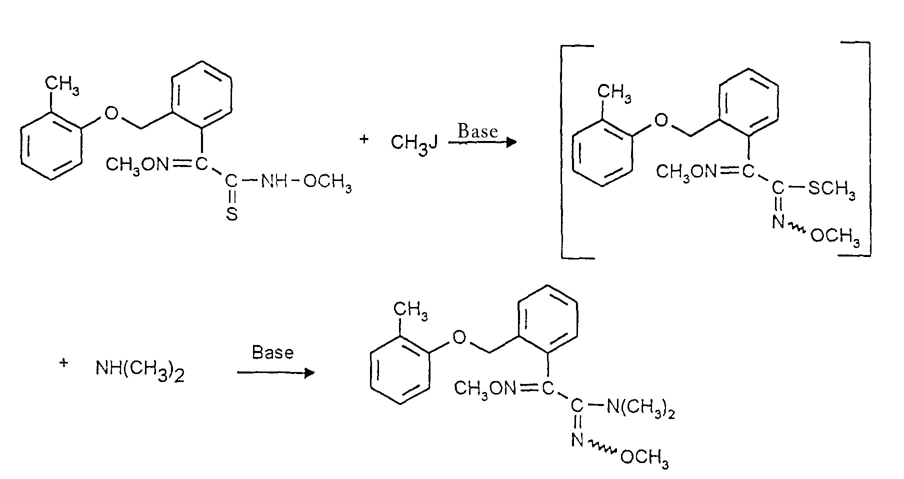

Die zur Durchführung des erfindungsgemäßen Verfahrens (a) als Ausgangsstoffe benötigten Thiocarbonyl-Derivate sind durch die Formel (II) allgemein definiert. In der Formel (II) haben E, G, X¹ und Z vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für diese Substituenten genannt wurden.

Die Thiocarbonyl-Derivate der Formel (II) sind bekannt (vgl. z.B. EP-A 432 503) bzw. nach in der Literatur beschriebenen Standardmethoden erhältlich, indem man beispielsweise die entsprechenden Keto-Derivate der allgemeinen Formel (VII) 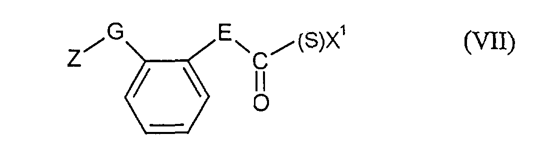 in welcher
- E, G, X¹ und Z: die oben angegebene Bedeutung haben,
mit einem Schwefelungsmittel, wie z.B. P₄S₁₀ oder Lawesson-Reagenz [2,4-Bis(4-methoxyphenyl)-1,3,2,4-dithiadiphosphetan-2,4-dithion], gegebenenfalls in einem Verdünnungsmittel, wie z.B. Xylol oder Toluol, bei Temperaturen zwischen 80 und 200°C umsetzt (vgl. auch die Herstellungsbeispiele).

Die Keto-Derivate der Formel (VII) sind bekannt bzw. nach bekannten Methoden erhältlich (vgl. hierzu z.B. die EP-A's: 253 213, 254 426, 299 694, 432 503, 460 575).

Die außerdem zur Durchführung der erfindungsgemäßen Verfahren (a) und (b) als Ausgangsstoffe benötigten Hydroxylamin-Derivate sind durch die Formel (III) allgemein definiert. In der Formel (III) hat Y¹ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für diesen Substituenten genannt wurden.

Die Hydroxylamin-Derivate der Formel (III) sowie deren Säureadditionssalze, wie beispielsweise deren Hydrochloride und Hydroacetate sind allgemein bekannte Verbindungen der organischen Chemie bzw. nach in der Literatur beschriebenen Standardmethoden erhältlich,

Die zur Durchführung des erfindungsgemäßen Verfahrens (b) als Ausgangsstoffe benötigten Amid-Derivate sind durch die Formel (IV) allgemein definiert. In der Formel (IV) haben E, G, X³ und Z vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für diese Substituenten genannt wurden.

Die Amid-Derivate der Formel (IV) sind noch nicht aus der Literatur bekannt. Sie sind jedoch nach in der Literatur beschriebenen Standardmethoden erhältlich, indem man beispielsweise die entsprechenden Keto-Derivate der allgemeinen Formel (VIII) 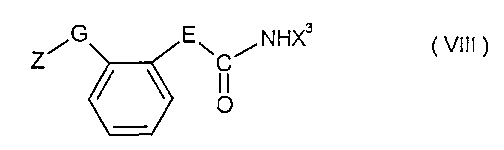 >
in welcher
- E, G, X³ und Z: die oben angegebene Bedeutung haben,
mit einem Schwefelungsmittel, wie z.B. P₄S₁₀ oder Lawesson-Reagenz [2,4-Bis(4-methoxyphenyl)-1,3,2,4-dithiadiphosphetan-2,4-dithion],gegebenenfallsineinem Verdünnungsmittel, wie z.B. Xylol oder Toluol, bei Temperaturen zwischen 80 und 200°C umsetzt (vgl. auch die Herstellungsbeispiele).

Die Keto-Derivate der Formel (VIII) sind bekannt bzw. nach bekannten Methoden erhältlich (vgl. hierzu z.B. die WO-A 92/13 830), indem man z.B. Keto-Derivate der allgemeinen Formel (VII) mit entsprechenden Aminen umsetzt.

Als Alkylierungsmittel zur Durchführung der erfindungsgemäßen Verfahren (b) und (c) kommen übliche Reagenzien infrage, wie beispielsweise Alkylhalogenide, insbesondere Methylchlorid, Methylbromid und Methyljodid sowie Dialkylsulfate, insbesondere Dimethylsulfat. Die Alkylierungsmittel sind allgemein bekannte Verbindungen der organischen Chemie.

Die zur Durchführung des erfindungsgemäßen Verfahrens (c) als Ausgangsstoffe benötigten Amid-Derivate sind durch die Formel (V) allgemein definiert. In der Formel (V) haben Ar, E, G, Y¹ und Z vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für diese Substituenten genannt wurden.

Die Amid-Derivate der Formel (V) sind noch nicht aus der Literatur bekannt. Sie sind jedoch nach in der Literatur beschriebenen Standardmethoden erhältlich, indem man beispielsweise die entsprechenden Keto-Derivate der allgemeinen Formel (IX) 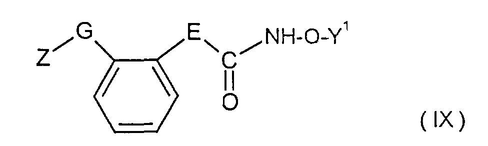 in welcher
- E, G, Y¹ und Z: die oben angegebene Bedeutung haben,
mit einem Schwefelungsmittel, wie z.B. P₄S₁₀ oder Lawesson-Reagenz [2,4-Bis(4-methoxyphenyl)-1,3,2,4-dithiadiphosphetan-2,4-dithion],gegebenenfallsineinem Verdünnungsmittel, wie z.B. Xylol oder Toluol, bei Temperaturen zwischen 80 und 200°C umsetzt.

Die Keto-Derivate der Formel (IX) sind noch nicht aus der Literatur bekannt,Sie sind jedoch nach in der Literatur beschriebenen Standardmethoden erhältlich, indem man beispielsweise Methyl-ester der Formel (VII) (X¹=CH₃) mit Hydroxylamin-Derivaten der Formel (III) gemäß Verfahren (a) umsetzt.

Als Verdünnungsmittel zur Durchführung der erfindungsgemäßen Verfahren (a) bis (c) kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäureamid, Ester, wie Essigsäureethylester oder Sulfoxide, wie Dimethylsulfoxid, Alkohole, wie Methanol oder Ethanol oder basische Lösungsmittel wie Pyridin oder Triethylamin.

Die erfindungsgemäßen Verfahren (a) und (b) werden vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -alkoholate, - carbonate oder -hydrogencarbonate, wie beispielsweise Natriumhydroxid, Natriummethylat, Natriumethylat, Kalium-t-butylat, Natriumcarbonat, Kaliumcarbonat oder Natriumhydrogencarbonat oder auch tertiäre Amine, wie beispielsweise Triethylamin, N,N-Deimethylanilin, Pyridin, N,N-Dimethylaminopyridin, Diazabicyclooctan (DABCO), Diazabicyclononen (DBN) oder Diazabicycloundecen (DBU). Auch saure Reaktionshilfsmittel wie beispielsweise p-Toluolsulfonsäure sind gegebenenfalls von Vorteil.

Die Reaktionstemperaturen können bei der Durchführung der erfindungsgemäßen Verfahren (a) bis (c) in einem größeren Bereich variiert werden. Im allgemeinen arbeitet man bei Temperaturen zwischen 0°C und + 200°C, vorzugsweise bei Temperaturen zwischen 20°C und 150°C.

Zur Durchführung der erfindungsgemäßen Verfahren (a) bis (c) setzt man pro Mol an Thiocarbonyl-Derivat der Formel (II) bzw. Amid-Derivat der Formel (IV) bzw. Amid-Derivat der Formel (V) im allgemeinen 1 bis 4 Mol, vorzugsweise 1 bis 2 Mol an Hydroxylamin-Derivat der Formel (III) bzw. Amin der Formel (VI) und gegebenenfalls 1 bis 3 Mol, vorzugsweise 1 bis 2 Mol an Reaktionshilfsmittel sowie gegebenenfalls 1 bis 10 Mol, vozugsweise 1 bis 5 Mol an Alkylierungsmittel ein.

Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen Sphaerotheca-, Podosphaera- und Venturie-Arten, zur Bekämpfung von Getreidekrankheiten, wie beispielsweise gegen Erysiphe-Arten, oder zur Bekämpfung von Reiskrankheiten, wie beispielsweise gegen Pyricularia oryzae eingesetzt werden. Daneben besitzen die erfindungsgemäßen Wirkstoffe eine gute in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, ChlorethyTene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Für die Mischungen kommen beispielsweise infrage:

### Fungizide:

2-Aminobutan;2-Anilino-4-methyl-6-cyclopropyl-pyrimidin;2',6'-Dibromo-2-methyl-4'-trifluoromethoxy-4'-trifluoro-methyl-1,3-thiazole-5-carboxanilic;2,6-Dichloro-N-(4-trifluoromethylbenzyl)benzamid;(E)-2-Methoxyimino-N-methyl-2-(2-phenoxyphenyl) acetamid; 8-Hydroxyquinolinsulfat; Methyl-(E)-2-{2-[6-(2-cyanophenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol, Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox, Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung, Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol, Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemeton M, Oxydeprofos,
Parathion A, Parathion M, Permethrin, Phenthoat, Phorat, Phosalon, Phosmet, Phosphamon, Phoxim, Pirimicarb, Pirimiphos M, Pirimiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1

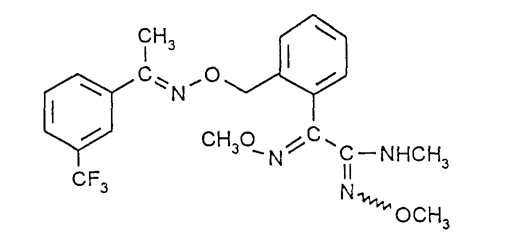

### (Verfahren b)

1,4g (3,3 m Mol) {2-[(1-(3-Trifluormethylphenyl)ethyliden)-aminooxymethyl]-phenyl}-2-methoximino-thioessigsäuremethylamidwerdenin 10ml Dimethylformamid mit 1,5g (10,8 m Mol) Kaliumcarbonat und 2g (14 m Mol) Methyljodid 3 Stunden bei 40°C gerührt. Zu dieser Mischung gibt man ein Gemisch aus 1,1g (13,1 m Mol) 0-Methylhydroxylammonium chlorid in 5ml Methanol und 1,8ml 2-molarer Natriummethylatlösung und erhitzt 30 Minuten unter Rückfluß. Anschließend gießt man das Reaktionsgemisch auf Wasser, extrahiert mit Diethylether und chromatographiert nach dem Abziehen des Lösungsmittels den Rückstand in Diethylether : Petrolether = 1:1.

Man erhält 0,7g (48,5% der Theorie) 1,2-Bis(methoximino)-1-methylamino-2-{2-[(1-(3-Trifluormethylphenyl)ethyliden)-aminooxymethyl]-phenyl}-ethan.
¹H-NMR (CDCl₃/Tetramethylsilan): 100 δ=2,283 (3H); 2,824 / 2,842 (3H); 3,788 (3H); 3,978 (3H); 5,019 / 5,037 (1H); 5,215 (2H); 7,2-7,6 (6H); 7,757 / 7,784 (1H); 7,847 (1H) ppm.

### Herstellung des Ausgangsproduktes

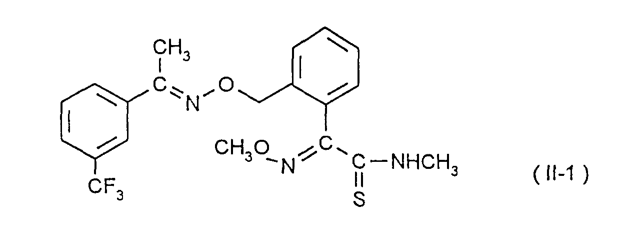

3g (7,36 m Mol) {2-[1-(3 -Trifluormethylphenyl)ethyliden)-aminooxymethyl]phenyl}-2-methoximino-essigsäuremethylamid werden mit 0,4g P₄S₁₀ in 30ml Toluol 15 Minuten lang unter Rückfluß erhitzt. Man filtriert ab, engt ein und chromatographiert den Rückstand in Diethylether : Petrolether = 1:1.

Man erhält 2g (64% der Theorie) {2-[1-(3-Trifluormethylphenyl)ethyliden)-aminooxymethyl]phenyl} -2-methoximino-thioessigsäure-methylamid.
¹H-NMR (CDCl₃/Tetramethylsilan): 100 δ = 2,222 (3H); 3,207 / 3,224 (3H); 3,956 (3H); 5,125 (2H); 7,0-8,0 (8H); 8,65 (1H) ppm.

### Beispiel 2

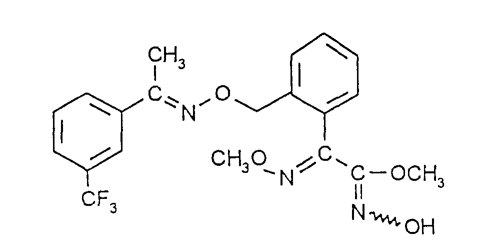

### (Verfahren a)

Zu 0,93g (13,4 m Mol) Hydroxylammoniumchlorid in 7ml Methanol tropft man 6,7ml 2-molare Natriummethylat-Lösung zu. Man gibt 4,5g (10,6 m Mol) {2-[(1-(3-Trifluormethylphenyl)ethyliden)-aminooxymethyl]-phenyl}-2-methoximino-thioessigsäure-methylester zu und erhitzt 15 Minuten lang unter Rückfluß. Man läßt das Reaktinsgemisch 24 Stunden bei Raumtemperatur stehen, zieht das Methanol im Vakuum ab und verteilt den Rückstand zwischen Wasser und Essigsäureethylester. Nach dem Abziehen des Lösungsmittels chromatographiert man in Diethylether Petrolether = 1:1.

Man erhält 2,7g (60,1% der Theorie) 1-Hydroximino-1-methoxy-2-methoximino-2-{2-[(1-(3-Trifluormethylphenyl)ethyliden)-aminooxymethyl]-phenyl}ethan.
¹H-NMR (CDCl₃/Tetramethylsilan): 100 δ =2,199 (3H); 3,949 (3H); 4,003 (3H); 5,114 (2H); 7,0-7,6 (6H); 7,7-7,9 (2H) ppm.

### Herstellung des Ausgangsprodukts

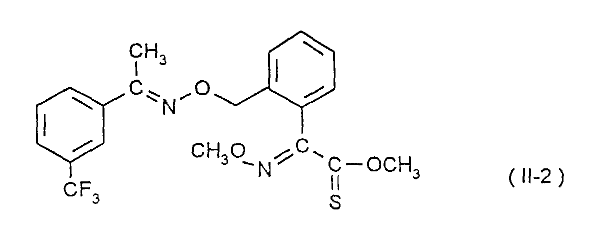

10g (0.024 Mol) {2-[(1-(3-Trifluormethylphenyl)ethyliden)-aminooxymethyl]-phenyl}-2-methoximino-essigsäuremethylester werden in 50ml Xylol mit 14,9g (0.036 Mol) Lawesson Reagenz 16 Stunden unter Rückfluß erhitzt. Danach gibt man nochmals 14,9g (0,036 Mol) Lawesson Reagenz zu und erhitzt weitere 16 Stunden unter Rückfluß. Man engt ein und chromatographiert den Rückstand in Petrolether tert. Butylmethylether = 4:1.

Man erhält 4,2g (40,4% der Theorie)(2-[(1-(3-Trifluormethylphenyl)ethyliden)-aminooxymethyl]-phenyl}-2-methoximino-thioessigsäure-methylester.
GC/MS : M= 424, 393, 362, 345, 317, 268, 240, 222, 208, 186, 145, 116, 89, 75, 47.

### Beispiel 3

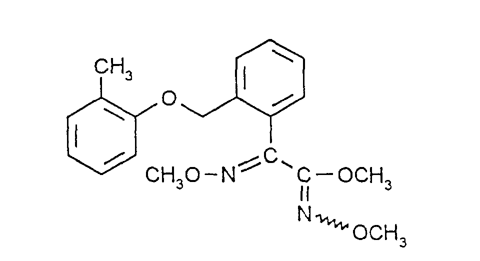

### (Verfahren a)

2g (0,024 Mol) 0-Methylhydroxylammoniumchlorid werden in 12ml Methanol mit 12ml einer 2-molaren Lösung von Natriummethylat in Methanol versetzt. Zu dieser Lösung gibt man 8,0g (0,024 Mol) [2-(2-Methylphenoxymethyl)-phenyl]-2-methoximino-thioessigsäuremethylester und erhitzt 15 Minuten unter Rückfluß. Man läßt das Reaktionsgemisch 24 Stunden bei Raumtemperatur stehen, zieht das Methanol im Vakuum ab und verteilt den Rückstand zwischen Wasser und Essigsäureethylester. Nach dem Abziehen des Lösungsmittels chromatographiert man in Diethylether : Petrolether = 1:3.

Man erhält 2,1g (25,5% der Theorie) 1-Methoxy-1,2-bis (methoximino)-2-(2-methylphenoxymethyl)phenyl-ethan.
¹H-NMR (CDCl₃/Tetramethylsilan): 100 δ =2,288 (3H); 3,761 (3H); 3,968 (3H); 3,980 (3H); 5,005 (2H); 6,783/6,811/6,842/6,866 (2H); 7,05-7,2 (2H); 7,3-7,5 (2H); 7,5-7,6 (1H) ppm.

Analog den Herstellungsbeispielen sowie entsprechend den allgemeinen Beschreibungen der erfindungsgemäßen Verfahren können beispielsweise die in der nachstehenden Tabelle 1 aufgeführten Verbindungen der Formel (I) hergestellt werden:

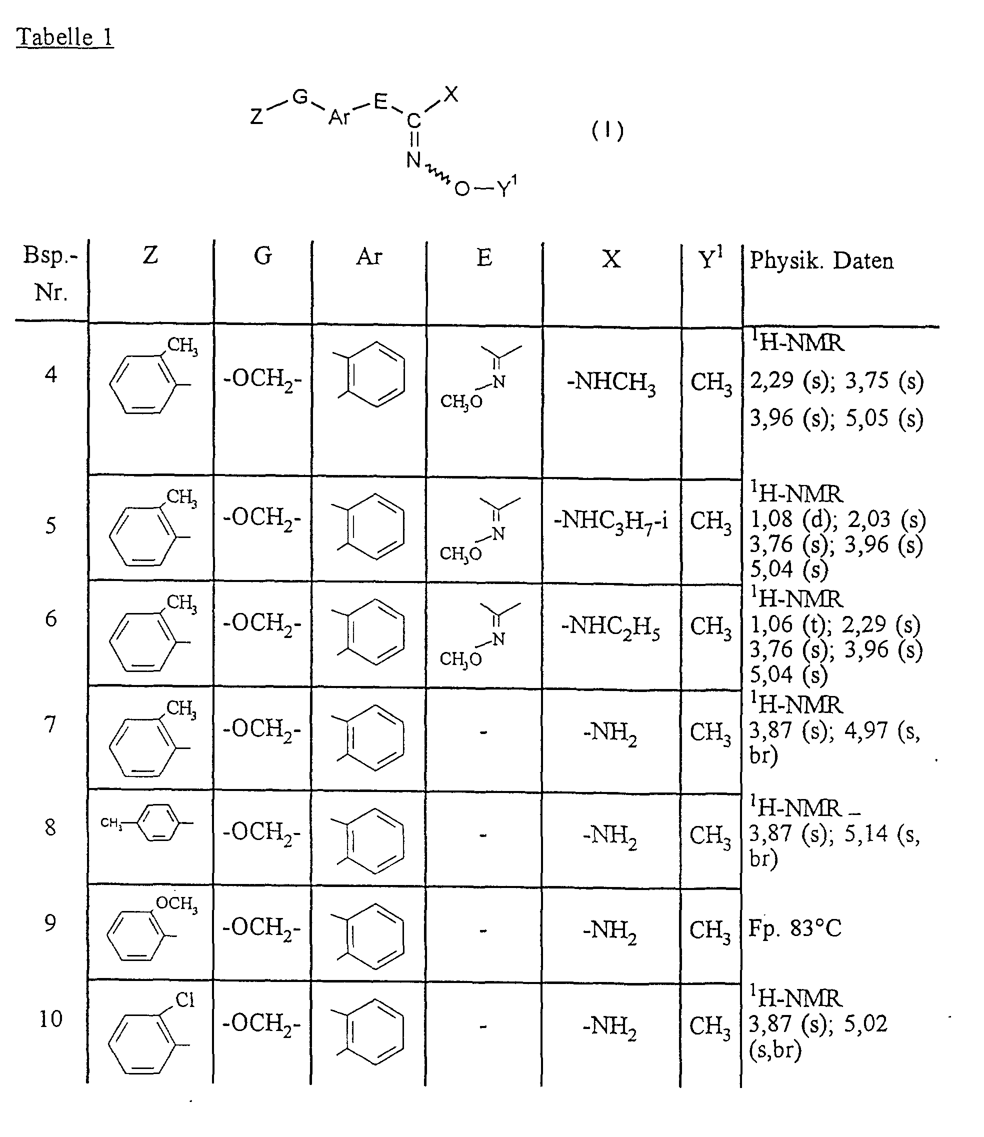

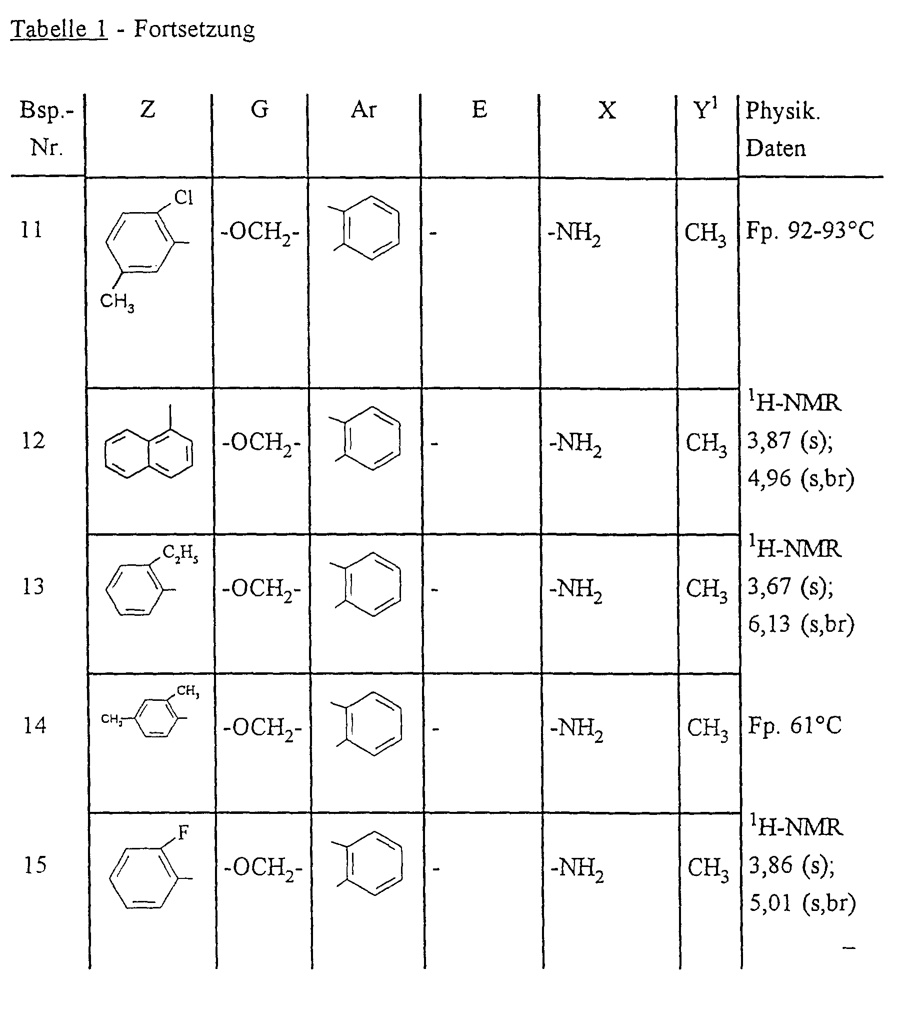

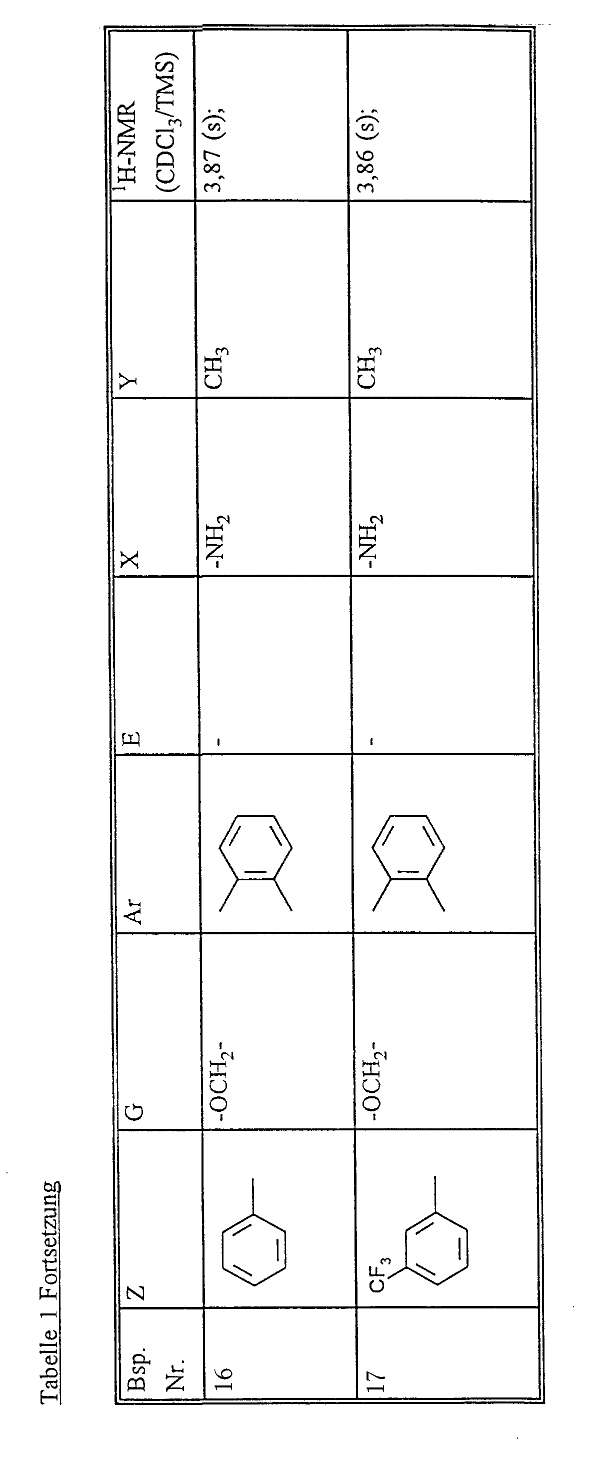

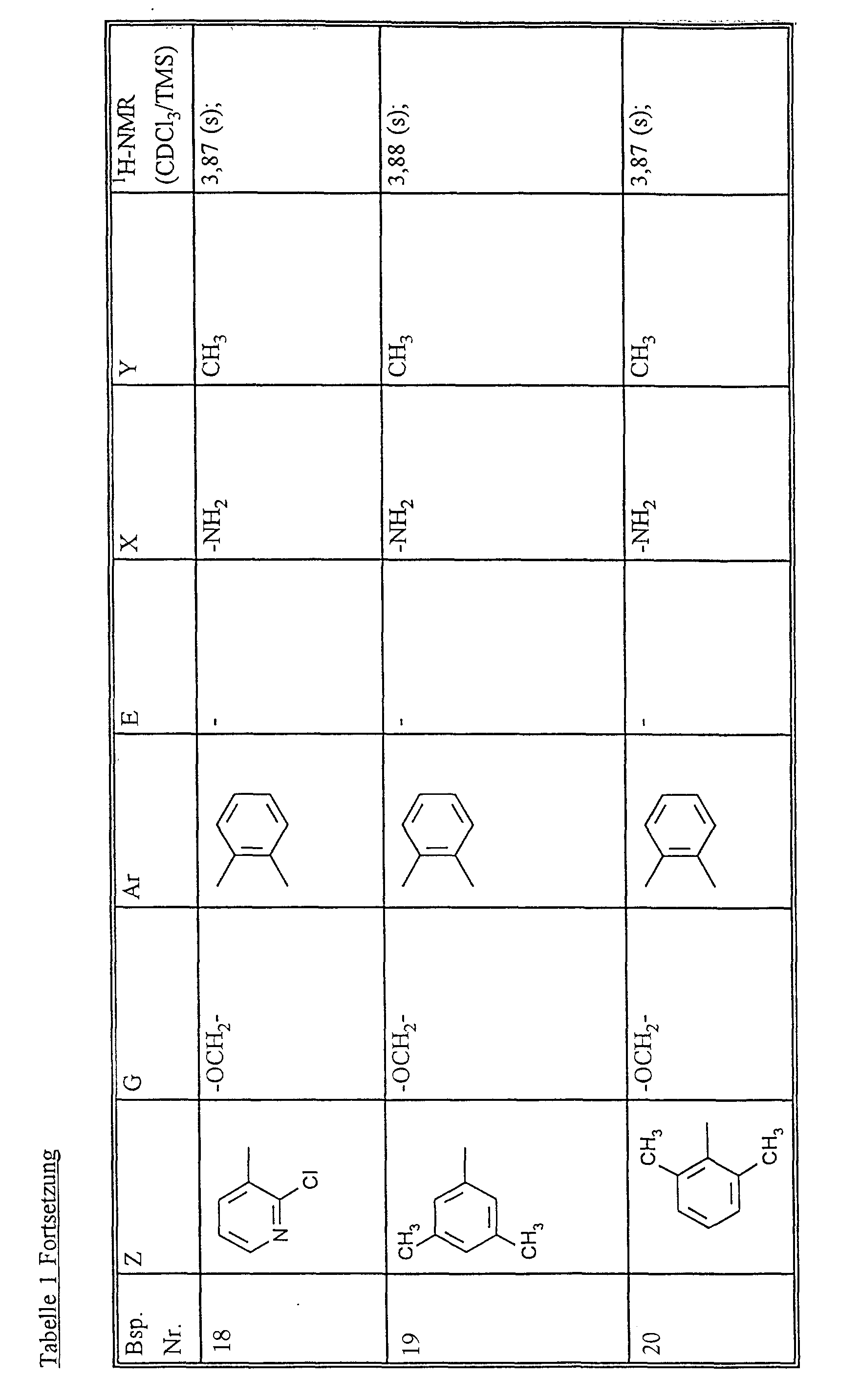

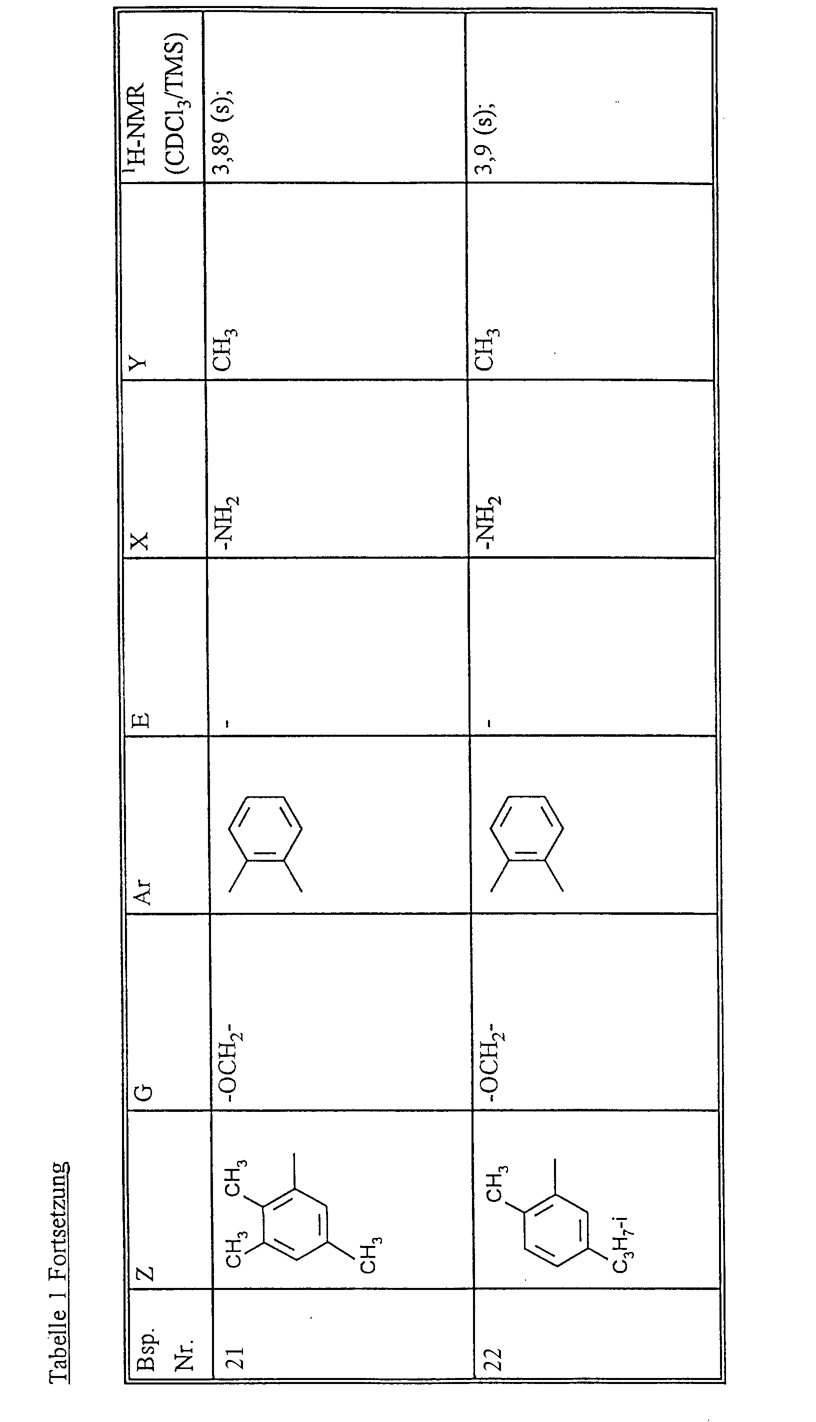

### Anwendungsbeispiele

### Beispiel A

| **Pyricularia-Test (Reis) /protektiv** | |
|---|---|
| Lösungsmittel | 12,5 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% relativer Luftfeuchtigkeit und 25°C aufgestellt.

4 Tage nach der Inokulation erfolgt die Auswertung des Krankheitsbefalls.

In diesem Test zeigt beispielsweise die Verbindung gemäß Herstellungsbeispiel 4 bei einer Wirkstoffkonzentration von 0,025 % einen Wirkungsgrad von 89%.

### Beispiel B

| **Erysiphe-Test (Weizen) /kurativ** | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. tritici bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

In diesem Test zeigt beispielsweise die Verbindung gemäß Herstellungsbeispiel 4 bei einer Wirkstoffkonzentration von 250g/ha einen Wirkungsgrad von 100%.

### Beispiel C

| **Erysiphe-Test (Gerste) /kurativ** | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

In diesem Test zeigt beispielsweise die Verbindung gemäß Herstellungsbeispiel 4 bei einer Wirkstoffkonzentration von 250g/ha einen Wirkungsgrad von 100%.

### Beispiel D

| **Erysiphe-Test (Weizen) /protektiv** | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel Emulgator und verdünnt das Konzentrat mit Wasser auf die gewünschte Konzentration.

Zur Prüfung auf protektive Wirksamkeit besprüht man junge Pflanzen der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele:

In diesem Test zeigt beispielsweise die Verbindung gemäß Herstellungsbeispiel 4 bei einer Wirkstoffkonzentration von 250g/ha einen Wirkungsgrad von 100%.

### Beispiel E

| **Erysiphe-Test (Gerste) /protektiv** | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel Emulgator und verdünnt das Konzentrat mit Wasser auf die gewünschte Konzentration.

Zur Prüfung auf protektive Wirksamkeit besprüht man junge Pflanzen der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele:

In diesem Test zeigt beispielsweise die Verbindung gemäß Herstellungsbeispiel 4 bei einer Wirkstoffkonzentration von 250g/ha einen Wirkungsgrad von 100%.

### Beispiel F

| **Sphaerotheca-Test (Gurke)** / **protektiv** | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigen z.B. die Verbindungen gemäß der Herstellungsbeispiele (2), (4) und (11) bis zu 100%ige Wirkungsgrade.

### Beispiel G

| **PodosphaeraTest (Apfel) / protektiv** | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70 % aufgestellt.

9 Tage nach der Inokulation erfolgt die Auswertung.

In diesem Test zeigen z.B. die Verbindungen gemäß der Herstellungsbeispiele (2), (4), und (11) bis zu 100%ige Wirkungsgrade.

### Beispiel H

| **Venturia-Test (Apfel) / protektiv** | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigen z.B. die Verbindungen gemäß der Herstellungsbeispiele (2), (4) und (11) bis zu 100%ige Wirkungsgrade.

## Patentansprüche

1. Verbindungen der Formel (I), 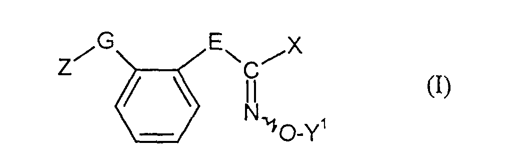 in welcher
E für eine direkte Bindung oder für eine Gruppierung der Formel 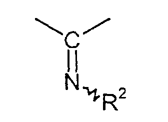 steht,
worin
R² für Methoxy steht,
G für eine Gruppe der Formel
-CH₂-O-,
oder
-O-CH₂-
steht,
X für die Gruppierungen -OX¹, -SX¹, -SOX¹, -SO₂X¹ oder -NX²X³ steht, wobei
X¹, X² und X³ unabhängig voneinander für Wasserstoff, für gegebenenfalls durch Fluor, Chlor, Cyano, Hydroxy, Amino, Methoxy, Methylthio (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls durch Fluor, Chlor, Brom, Cyano, Carboxy, Phenyl (welches gegebenenfalls durch Fluor, Chlor, Methyl, Trifluormethyl oder Trifluormethoxy substituiert ist), Methyl, Ethyl, n- oder i-Propyl, Methoxycarbonyl oder Ethoxycarbonyl substituiertes Cyclopropyl, Cyclobutyl, Cyclopentyl oder Cyclohexyl,
für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy, oder
X² und X³ zusammen einen gegebenenfalls wie oben substituierten Pyrazol-, Imidazol- oder Triazolring bilden,
Y¹ für Wasserstoff, für gegebenenfalls durch Fluor, Chlor, Cyano, Hydroxy, Amino, Methoxy, Methylthio (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls durch Fluor, Chlor, Brom, Cyano, Carboxy, Phenyl (welches gegebenenfalls durch Fluor, Chlor, Methyl, Trifluormethyl oder Trifluormethoxy substituiert ist), Methyl, Ethyl, n- oder i-Propyl, Methoxycarbonyl oder Ethoxycarbonyl substituiertes Cyclopropyl, Cyclobutyl, Cyclopentyl oder Cyclohexyl,
für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy,
Z für gegebenenfalls substituiertes Phenyl oder gegebenenfalls substituiertes Pyridinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

2. Verfahren zur Herstellung von Verbindungen der Formel (I) gemäß Anspruch 1, **dadurch gekennzeichnet, daß** man
a) Thiocarbonyl-Derivate der allgemeinen Formel (II), 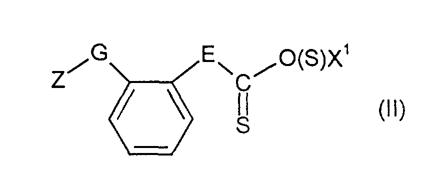 in welcher
E, G, X¹ und Z die in Anspruch 1 angegebene Bedeutung haben,
mit Hydroxylamin-Derivaten der allgemeinen Formel (III),
H₂N―O―Y¹ (III)
in welcher
Y¹ die oben angegebene Bedeutung hat,
oder mit deren Säureadditionssalzen gegebenenfalls in Gegenwart eines Verdünnungsmittels sowie gegebenenfalls in Gegenwart eines Reaktionshilfsmittels umsetzt;
oder
b) Amid-Derivate der allgemeinen Formel (IV), 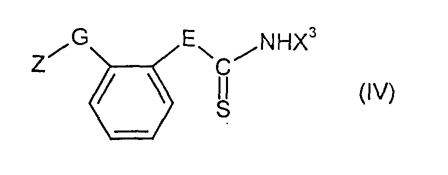 in welcher
E, G, X³ und Z die in Anspruch 1 angegebenen Bedeutungen haben,
in üblicher Weise alkyliert und die so erhaltenen Imino-Derivate der Formel (IVa), 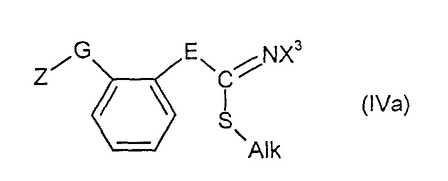 in welcher
E, G, X³ und Z die in Anspruch 1 angegebenen Bedeutungen haben,
und
Alk für Alkyl steht,
gegebenenfalls ohne Isolierung mit Hydroxylamin-Derivaten der allgemeinen Formel (III) oder mit deren Säureadditionssalzen gegebenenfalls in Gegenwart eines Verdünnungsmittels sowie gegebenenfalls in Gegenwart eines Reaktionshilfsmittels umsetzt;
oder
c) Amid-Derivate der allgemeinen Formel (V), 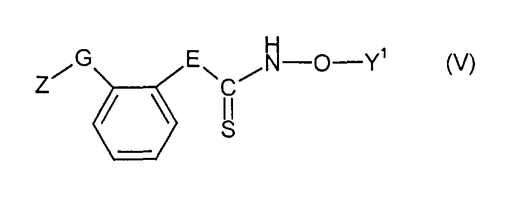 in welcher
E, G, Y¹ und Z die in Anspruch 1 angegebenen Bedeutungen haben,
in üblicher Weise alkyliert und die so erhaltenen Oxime der Formel (Va), 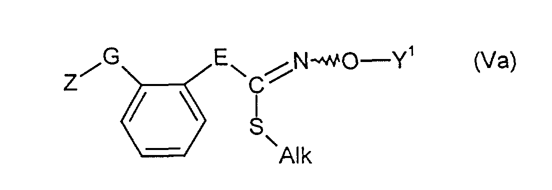 in welcher
E, G, Y¹, Z und Alk die oben angegebenen Bedeutungen haben,
gegebenenfalls ohne Isolierung mit Aminen der allgemeinen Formel (VI),
NHX²X³ (VI)
in welcher
X² und X³ die oben angegebenen Bedeutungen haben,
gegebenenfalls in Gegenwart eines Verdünnungsmittels umsetzt.

3. Mikrobizide Mittel, **gekennzeichnet durch** einen Gehalt an mindestens einer Verbindung der Formel (I) gemäß Anspruch 1 neben Streckmitteln und/oder oberflächenaktiven Stoffen.

4. Verwendung von Verbindungen der Formel (I) gemäß Anspruch 1 zur Bekämpfung unerwünschter Mikroorganismen.

5. Verfahren zur Bekämpfung unerwünschter Mikroorganismen, **dadurch gekennzeichnet, dass** man Verbindungen der Formel (I) gemäß Anspruch 1 auf die Mikroorganismen und/oder deren Lebensraum ausbringt.

6. Verfahren zur Herstellung von mikrobiziden Mitteln, **dadurch gekennzeichnet, dass** man Verbindungen der Formel (I) gemäß Anspruch 1 mit Streckmitteln und/oder oberflächenaktiven Mitteln vermischt.

## Claims

1. Compounds of the formula (I), 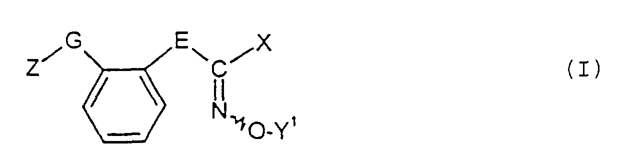 in which
E represents a direct bond or a grouping of the formula 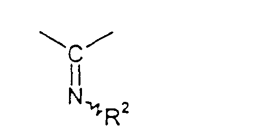 in which
R² represents methoxy,
G represents a group of the formula
-CH₂-O-,
or
-O-CH₂-
X represents the groupings -OX¹, -SX¹, -SOX¹, -SO₂X¹ or -NX²X³, wherein
X¹, X² and X³, independently of one another, represent hydrogen, or methyl, ethyl, n- or i-propyl, n, i, s- or t-butyl optionally substituted by fluorine, chlorine, cyano, hydroxy, amino, methoxy, methylthio (which may each be optionally substituted by fluorine and/or chlorine), cyclopropyl, cyclobutyl, cyclopentyl or cyclohexyl, each optionally substituted by fluorine, chlorine, bromine, cyano, carboxy, phenyl (which is optionally substituted by fluorine, chlorine, methyl, trifluoromethyl or trifluoromethoxy), methyl, ethyl n- or i-propyl, methoxycarbonyl or ethoxycarbonyl,
phenyl, pyridinyl, pyrimidinyl, pyridazinyl, pyrazinyl, 1,2,3-triazinyl, 1,2,4-triazinyl or 1,3,5-triazinyl, each optionally substituted, wherein the possible substituents are chosen from the following list:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulfinyl, ethylsulfinyl, methylsufonyl or ethylsufonyl, trifluoromethyl, difluoromethoxy, trifluoromethoxy, difluoromethylthio, trifluoromethylthio, trifluoromethylsulfinyl or trifluoromethylsulfonyl, methoxycarbonyl, ethoxycarbonyl, methoximinomethyl, ethoximinomethyl, methoximinoethyl, ethoximinoethyl, each being optionally substituted once or several times, identically or differently, by fluorine, chlorine, methyl or ethyl substituted methylenedioxy or ethylenedioxy, and also phenyl, phenoxy, benzyl or benzyloxy, each being optionally substituted in the phenyl section, once or several times, identically or differently, by fluorine, chlorine, bromine, cyano, methyl, ethyl, n- or i-propyl, n-, i-, s- or t-butyl, trifluoromethyl, methoxy, ethoxy, n- or i-propoxy; difluoromethoxy or trifluoromethoxy, or
X² and X³ together form a pyrazole, imidazole or triazole ring, optionally substituted as above
Y¹ represents hydrogen or methyl, ethyl, n- or i-propyl, n, i-, s- or t-butyl optionally substituted by fluorine, chlorine, cyano, hydroxy, amino, methoxy, methylthio (which may each be optionally substituted by fluorine and/or chlorine),
cyclopropyl, cyclobutyl, cyclopentyl or cyclohexyl, each optionally substituted by fluorine, chlorine, bromine, cyano, carboxy, phenyl (which is optionally substituted by fluorine, chlorine, methyl, trifluoromethyl or trifluoromethoxy), methyl, ethyl n- or i-propyl, methoxycarbonyl or ethoxycarbonyl,
phenyl, pyridinyl, pyrimidinyl, pyridazinyl, pyrazinyl, 1,2,3-triazinyl, 1,2,4-triazinyl or 1,3,5-triazinyl, each optionally substituted, wherein the possible substituents are chosen from the following list:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulfinyl, ethylsulfinyl, methylsufonyl or ethylsufonyl, trifluoromethyl, difluoromethoxy, trifluoromethoxy, difluoromethylthio, trifluoromethylthio, trifluoromethylsulfinyl or trifluoromethylsulfonyl, methoxycarbonyl, ethoxycarbonyl, methoximinomethyl, ethoximinomethyl, methoximinoethyl, ethoximinoethyl each optionally substituted once or several times, identically or differently by fluorine, chlorine, methyl or ethyl substituted methylenedioxy or ethylenedioxy, and also phenyl, phenoxy, benzyl or benzyloxy, each optionally substituted in the phenyl section, once or several times, identically or differently, by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy,
Z represents optionally substituted phenyl or optionally substituted pyridinyl, wherein the possible substituents are chosen from the following list:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulfinyl, ethylsulfinyl, methylsufonyl or ethylsufonyl, trifluoromethyl, difluoromethoxy, trifluoromethoxy, difluoromethylthio, trifluoromethylthio, trifluoromethylsulfinyl or trifluoromethylsulfonyl, methoxycarbonyl, ethoxycarbonyl, methoximinomethyl, ethoximinomethyl, methoximinoethyl, ethoximinoethyl, each being optionally substituted once or several times, identically or differently, by fluorine, chlorine, methyl or ethyl substituted methylenedioxy or ethylenedioxy, and also phenyl, phenoxy, benzyl or benzyloxy, each being optionally substituted in the phenyl section, once or several times, identically or differently, by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy.

2. A process for preparing compounds of the formula (I) according to Claim 1, **characterised in that**
a) thiocarbonyl derivatives of the general formula (II) 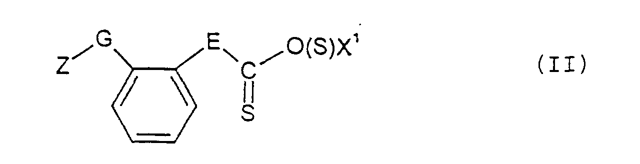 in which
E, G, X¹ and Z are defined in the same way as in Claim 1,
are reacted with hydroxylamine derivatives of the general formula (III)
H₂N-O-Y¹ (III)
in which
Y¹ is defined in the same way as given above,
or with their acid addition salts optionally in the presence of a diluent and optionally in the presence of a reaction auxiliary substance;
or
b) amide derivatives of the general formula (IV), 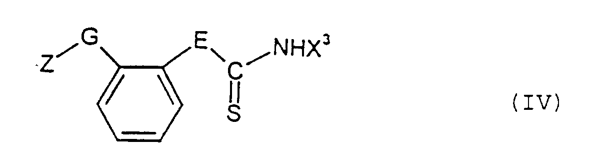 in which
E, G, X³ and Z are defined in the same way as in Claim 1,
are alkylated in a conventional manner and the imino derivatives thus obtained of the formula (IVa), 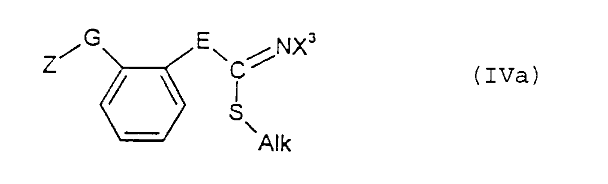 in which
E, G, X³ and Z are defined in the same way as in Claim 1,
and
Alk represents alkyl,
optionally without isolation, are reacted with hydroxylamine derivatives of the general formula (III) or with their acid addition salts optionally in the presence of a diluent and optionally in the presence of a reaction auxiliary substance;
or
c) amide derivatives of the general formula (V), 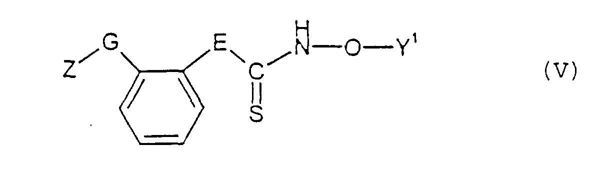 in which
E, G, Y¹ and Z are defined in the same way as in Claim 1,
are alkylated in a conventional manner and the oximes thus obtained of the formula (Va) 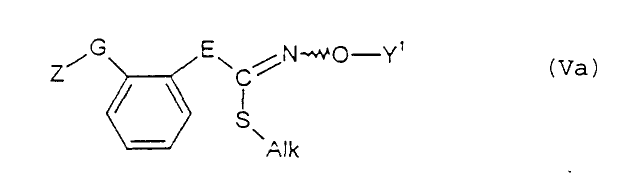 in which
E, G, Y¹, Z and Alk are defined in the same way as above,
optionally without isolation, are reacted with amines of the general formula (VI)
NHX²X³ (VI)
in which
X² and X³ are defined in the same way as above,
optionally in the presence of a diluent.

3. A microbicidal agent, **characterised by** a concentration of at least one compound of the formula (I) according to Claim 1 in addition to extenders and/or surface-active substances.

4. The use of compounds of the formula (I) according to Claim 1 for controlling unwanted microorganisms.

5. A process for controlling unwanted microorganisms, **characterised in that** compounds of the formula (I) according to Claim 1 are applied to the microorganisms and/or to their habitat.

6. A process for preparing microbicidal agents, **characterised in that** compounds of the formula (I) according to Claim 1 are blended with extenders and/or surface-active agents.

## Revendications

1. Composés de formule (I) 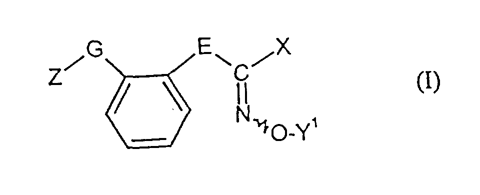 dans laquelle
E est une liaison directe ou représente un groupement de formule 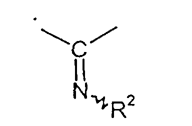 dans laquelle
R² est un groupe méthoxy,
G est un groupe de formule
-CH₂-O-,
ou
-O-CH₂-,
X représente les groupements -OX¹, -SX¹, -SOX¹, -SO₂X¹ ou -NX²X³, où
X¹, X² et X³ représentent, indépendamment les uns des autres, l'hydrogène, un groupe méthyle éventuellement substitué par du fluor, du chlore, un radical cyano, hydroxy, amino, méthoxy, méthylthio (chacun étant substitué éventuellement par du fluor et/ou du chlore), un groupe éthyle, n-propyle, isopropyle, n-butyle, isobutyle, sec.-butyle ou tertio-butyle, un groupe cyclopropyle, cyclobutyle, cyclopentyle ou cyclohexyle dont chacun est éventuellement substitué par du fluor, du chlore, du brome, un radical cyano, carboxy, phényle (qui est substitué, le cas échéant, par du fluor, du chlore, un radical méthyle, trifluorométhyle ou trifluorométhoxy), méthyle, éthyle, n-propyle, isopropyle, méthoxycarbonyle ou éthoxycarbonyle,
un groupe phényle, pyridinyle, pyrimidinyle, pyridazinyle, pyrazinyle, 1,2,3-triazinyle, 1,2,4-triazinyle ou 1,3,5-triazinyle dont chacun est éventuellement substitué, les substituants possibles étant choisis dans l'énumération suivante :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propoylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhyl-sulfonyle, méthoxycarbonyle, éthoxycarbonyle, méthoximinométhyle, éthoximinométhyle, méthoximinoéthyle, éthoximino-éthyle, méthylènedioxy ou éthylènedioxy portant chacun, le cas échéant, un ou plusieurs substituants fluoro, chloro, méthyle ou éthyle identiques ou différents, ainsi que phényle, phénoxy, benzyle ou benzyloxy portant chacun, le cas échéant, dans la partie phényle un ou plusieurs substituants, identiques ou différents, fluoro, chloro, bromo, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy, ou bien
X² et X³ forment ensemble un noyau pyrazole, imidazole ou triazole éventuellement substitué comme ci-dessus,
Y¹ représente l'hydrogène, un groupe méthyle éventuellement substitué par du fluor, du chlore, un radical cyano, hydroxy, amino, méthoxy, méthylthio (chacun étant substitué, le cas échéant, par du fluor et/ou du chlore), un groupe éthyle, n-propyle, isopropyle, n-butyle, isobutyle, sec.-butyle ou tertio-butyle,
un groupe cyclopropyle, cyclobutyle, cyclopentyle ou cyclohexyle portant chacun, le cas échéant, un substituant fluoro, chloro, bromo, cyano, carboxy, phényle (qui est substitué, le cas échéant, par un radical fluoro, chloro, méthyle, trifluorométhyle ou trifluorométhoxy), méthyle, éthyle, n-propyle, isopropyle, méthoxycarbonyle ou éthoxycarbonyle,
un groupe phényle, pyridinyle, pyrimidinyle, pyridazinyle, pyrazinyle, 1,2,3-triazinyle, 1,2,4-triazinyle ou 1,3,5-triazinyle dont chacun est éventuellement substitué, les substituants possibles étant choisis dans l'énumération suivante :
fluor, chlore, bromo, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthoxycarbonyle, éthoxycarbonyle, méthoximinométhyle, éthoximinométhyle, méthoximinoéthyle, éthoximino-éthyle, un groupe méthylènedioxy ou éthylènedioxy portant chacun, le cas échéant, un ou plusieurs substituants, identiques ou différents, fluoro, chloro, méthyle ou éthyle, ainsi qu'un groupe phényle, phénoxy, benzyle ou benzyloxy portant éventuellement dans chaque cas, dans la partie phényle, un ou plusieurs substituants, identiques ou différents, fluoro, chloro, bromo, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy,
Z est un groupe phényle éventuellement substitué ou un groupe pyridinyle éventuellement substitué, les substituants possibles étant choisis dans l'énumération suivante :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthoxycarbonyle, éthoxycarbonyle, méthoximinométhyle, éthoximinométhyle, méthoximinoéthyle, éthoximino-éthyle, méthylènedioxy ou éthylènedioxy portant chacun, le cas échéant, un ou plusieurs substituants fluoro, chloro, méthyle ou éthyle identiques ou différents, ainsi que phényle, phénoxy, benzyle ou benzyloxy portant chacun, le cas échéant, dans la partie phényle, un ou plusieurs substituants, identiques ou différents, fluor, chlore, brome, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy.

2. Procédé de production de composés de formule (I) suivant la revendication 1, **caractérisé en ce que**
a) on fait réagir des dérivés de thiocarbonyle de formule générale (II) 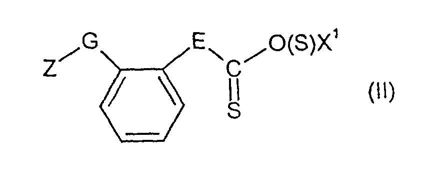 dans laquelle
E, G, X¹ et Z ont la définition indiquée dans la revendication 1,
avec des dérivés d'hydroxylamines de formule générale (III)
H₂N―O―Y¹ (III)
dans laquelle
Y¹ a la définition indiquée ci-dessus,
ou avec leurs sels d'addition d'acides, le cas échéant en présence d'un diluant de même que le cas échéant en présence d'un auxiliaire de réaction ;
ou bien
b) on alkyle d'une manière classique des dérivés d'amides de formule générale (IV) 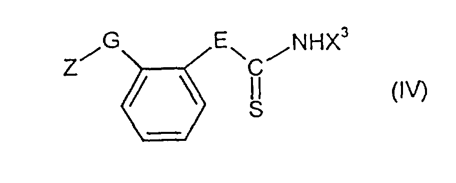 dans laquelle
E, G, X³ et Z ont les définitions indiquées dans la revendication 1,
et on fait réagir les dérivés imino ainsi obtenus de formule (IVa) 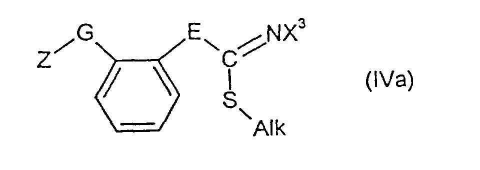
dans laquelle
E, G, X³ et Z ont les définitions indiquées dans la revendication 1, et
Alk est un groupe alkyle,
éventuellement sans isolement, avec des dérivés d'hydroxylamines de formule générale (III) ou avec leurs sels d'addition d'acides, éventuellement en présence d'un diluant de même qu'en présence éventuelle d'un auxiliaire de réaction ;
ou bien
c) on alkyle de manière classique des dérivés d'amides de formule générale (V) 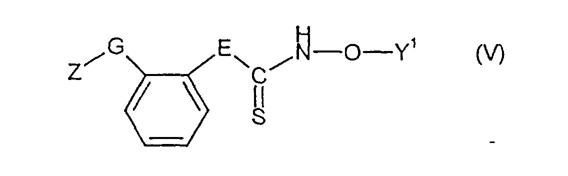 dans laquelle
E, G, Y¹ et Z ont les définitions données dans la revendication 1,
et on fait réagir les oximes ainsi obtenues de formule (Va) 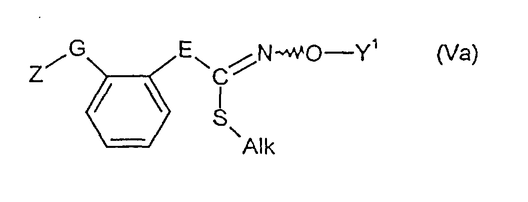 dans laquelle
E, G, Y¹, Z et Alk ont les définitions indiquées ci-dessus,
éventuellement sans isolement, avec des amines de formule générale (VI)
NHX²X³ (VI)
dans laquelle
X² et X³ ont les définitions indiquées ci-dessus,
éventuellement en présence d'un diluant.

3. Compositions microbicides, **caractérisées par** une teneur en au moins un composé de formule (I) suivant la revendication 1, à côté de diluants et/ou d'agents tensio-actifs.

4. Utilisation de composés de formule (I) suivant la revendication 1 pour combattre des micro-organismes indésirables.

5. Procédé pour combattre des micro-organismes indésirables, **caractérisé en ce qu'**on applique des composés de formule (I) suivant la revendication 1 sur les micro-organismes et/ou sur leur milieu.

6. Procédé de préparation de compositions microbicides, **caractérisé en ce qu'**on mélange des composés de formule (I) suivant la revendication 1 avec des diluants et/ou des agents tensio-actifs.
